# EUROPÄISCHE PATENTSCHRIFT

(11) **EP 1 565 713 B1**
(45) Veröffentlichungstag und Bekanntmachung des Hinweises auf die Patenterteilung: **30.07.2008**
(21) Anmeldenummer: 03812125.7
(22) Anmeldetag: 07.10.2003
(51) Int. Cl.: G01H 9/00, G01L 11/02, A61B 17/22

(54) **OPTISCHES HYDROPHON ZUM MESSEN DER SCHALLDRUCKVERTEILUNG IN EINEM FLUIDEN MEDIUM**
OPTICAL HYDROPHONE FOR DETERMINING AN ACOUSTIC PRESSURE WAVE DISTRIBUTION IN A FLUID MEDIUM& x9;
HYDROPHONE OPTIQUE POUR MESURER LA REPARTITION DE LA PRESSION ACOUSTIQUE DANS UN MILIEU FLUIDE

(30) Priorität: 29.11.2002 DE 10256077; 01.08.2003 DE 10335988
(43) Veröffentlichungstag der Anmeldung: 24.08.2005
(73) Patentinhaber: SIEMENS AKTIENGESELLSCHAFT, 80333 München (DE)
(72) Erfinder: GRANZ, Bernd, 90522 Oberasbach (DE); NANKE, Ralf, 91080 Spardorf (DE)
(86) Internationale Anmeldenummer: PCT/DE2003/003320
(87) Internationale Veröffentlichungsnummer: WO 2004/051203

(56) Entgegenhaltungen:
- DE-A- 4 304 139
- DE-A- 19 541 952
- DE-C- 19 741 747
- US-A- 5 315 364

## Beschreibung

Die Erfindung bezieht sich auf ein optisches Hydrophon zum Messen der Schalldruckverteilung in einem fluiden Medium, insbesondere zum Vermessen eines Ultraschall-Stoßwellenfeldes oder von diagnostischem Ultraschall.

Bei akustischen Stoßwellen, wie sie beispielsweise in der Lithotripsie verwendet werden, treten hohe Drücke bis etwa 10⁸ Pa mit Anstiegszeiten im Bereich von wenigen ns auf. Die Messung solcher hohen Drücke erfordert Sensoren mit einer hohen mechanischen Stabilität. Außerdem sollten diese Sensoren weitgehend miniaturisiert sein, um die Schalldruckverteilung in einem Stoßwellenfeld mit möglichst hoher örtlicher Auflösung vermessen zu können.

Aus der EP 0 354 229 B1 oder der DE 38 02 024 A1 sowie aus J. Staudenraus, W. Eisenmenger, "Fibre-optic probe hydrophone for ultrasonic and shock-wave measurements in water", Ultrasonics 1993, Vol.31, No.4, Seite 267-273, sind jeweils Messanordnungen bekannt, bei der zur Messung der räumlichen und zeitlichen Verteilung des Druckes von Ultraschall-Stoßwellen in einer Flüssigkeit das am freien Ende eines Lichtwellenleiters reflektierte Licht verwendet wird. Bei dieser bekannten faseroptischen Messanordnung wird ausgenutzt, dass die hohe Druckamplitude eine Dichteänderung und somit eine Änderung des Brechungsindex der Flüssigkeit in unmittelbarer Nähe des freien Endes erzeugt, die den Anteil des in den Lichtwellenleiter an der Grenzfläche zurückreflektierten Lichtes moduliert. Die zur Messung verwendeten Lichtwellenleiter haben dabei einen Durchmesser, der 0,1 mm nicht überschreitet. Das freie, die Reflektivität der Grenzfläche Flüssigkeit / Lichtwellenleiter bestimmende Ende des Lichtwellenleiters wird durch eine kugelförmige oder ebene, senkrecht zur Lichtwellenleiterachse stehende Endfläche gebildet. Durch die Kleinheit dieser Endfläche wird eine für die Messung von fokussierten Stoßwellen erforderliche hohe Ortsauflösung, geringe Richtungsempfindlichkeit und hohe Bandbreite erzeugt.

Aus der DE 39 32 711 A1 ist ein faseroptischer Stoßwellensensor bekannt, bei dem das freie Ende des Lichtwellenleiters als Rotationskörper gestaltet ist, dessen Hüllkurve sich durch ein Polynom dritten Grades beschreiben lässt. Durch diese Maßnahme sollen auch bei der Verwendung von Lichtwellenleitern mit größerem Durchmesser sowohl die Empfindlichkeit als auch die Ortsauflösung verbessert werden.

Aus Koch, Ch., "Coated fiber-optic hydrophone for ultrasonic measurement", Ultrasonics 34, 1996, Seite 687-689, ist ein faseroptisches Hydrophon bekannt, das sowohl die Änderungen des Brechungsindex des umgebenden Fluids als auch die Änderung der Eigenschaften eines an der Faserspitze durch dielektrische Schichten gebildeten Interferometers nutzt, um auf diese Weise die Empfindlichkeit der Messanordnung zu erhöhen.

Nachteilig an den bekannten faseroptischen Hydrophonen ist jedoch, dass diese sehr bruchempfindlich sind und bereits nach 10 bis 100 Stoßwellen bei etwa 50 MPa zerstört sein können. Darüber hinaus ist ein hoher fertigungstechnischer Aufwand erforderlich, um die freien Enden der Lichtwellenleiter reproduzierbar mit der jeweils erforderlichen Form herzustellen.

In der Literatur, beispielsweise in Beard, PC, Mills TN, "An optical detection system for biomedical photoacoustic imaging", Proc. SpiE 3916, 2000, Seite 100-109, oder Beard, PC, "Photoacoustic imaging of blood ressel equivalent phantoms", Proc. SpiE 4618, 2002, Seite 54-62, sind auch interferometrische Messanordnungen bekannt, bei denen ein Polymerfilm als großflächiger Fabry-Perot-Interferometer dient, das optisch punktweise abgetastet wird, so dass sich ein 2-dimensionales Bild der Schalldruckverteilung ergibt. Eine solche Vorrichtung ist jedoch für das Vermessen von Stoßwellen nicht geeignet.

Der Erfindung liegt nun die Aufgabe zugrunde, ein optisches Hydrophon zum Messen der Schalldruckverteilung in einem fluiden Medium anzugeben, das fertigungstechnisch einfach herzustellen ist, eine hohe Lebensdauer aufweist und dessen räumliches Auflösungsvermögen vergleichbar ist mit dem räumlichen Auflösungsvermögen, wie es durch die im Stand der Technik bekannten faseroptischen Hydrophone erzielt werden kann.

Die genannte Aufgabe wird gemäß der Erfindung gelöst mit einem optischen Hydrophon mit den Merkmalen des Patentanspruchs 1. Ein solches optisches Hydrophon umfasst eine Lichtquelle zum Erzeugen von Licht und zum Beleuchten eines an einer Grenzfläche zwischen einem optisch transparenten Körper und dem schallführenden Medium befindlichen Flächenbereiches sowie eine Messeinrichtung zum Messen der Intensität eines an diesem Flächenbereich reflektierten Lichtes als Maß für den Schalldruck, wobei der beleuchtete Flächenbereich kleiner ist als die zwischen dem Körper und dem Medium gebildete Grenzfläche.

Die Erfindung beruht dabei auf der Idee, dass es zum Erzielen einer hohen örtlichen Auflösung nur auf die Größe des an der Grenzfläche zwischen dem transparenten Körper und dem schallführenden Medium beleuchteten Flächenbereich ankommt. Mit anderen Worten: Der transparente Körper muss nicht zwingend als Lichtleiter gestaltet sein, in dem das Licht durch Reflexion an den Wänden geführt ist. Vielmehr ist es ausreichend, durch entsprechende Strahlformung einen Lichtstrahl zu erzeugen, der sich im transparenten Körper frei ausbreitet und durch entsprechende Strahlformung im Bereich der Grenzfläche einen an den jeweiligen Bedarfszweck angepassten Strahlquerschnitt aufweist. Auf diese Weise kann der transparente Körper Abmessungen aufweisen, die sehr viel größer als der Strahlquerschnitt sind, und kann dementsprechend massiv ausgeführt werden, so dass er durch Stosswellen, wie sie im Fokus eines Lithrotripters auftreten können, nicht mehr zerstört werden kann. Außerdem kann die Grenzfläche problemlos bearbeitet werden, so dass eine hohe Reproduzierbarkeit mit geringem fertigungstechnischen Aufwand erreichbar ist.

Aus G. Paltauf u. H. Schmidt-Kloiber, "Measurement of laserinduced acoustic waves with a calibrated optical transducer", J. Appl. Phys. 82 (4), 1997, S. 1525 - 1531, ist eine Messanordnung zum Messen der Absorption eines kurzen Laserimpulses in einer Probe bekannt, bei der die in der Probe durch den Laserstrahl erzeugten Schallwellen in eine mit Wasser gefüllte Kammer eingekoppelt werden und dort zu einer Modulation des Brechungsindex führen. Die Kammer grenzt an die Basisfläche eines Glasprismas, in das seitlich Licht eingekoppelt wird und sich dort frei, d. h. ohne Reflexion an Grenzflächen ausbreitet, und auf die Basisfläche unter dem Grenzwinkel der Totalreflexion auftrifft. Die durch die Modulation des Brechungsindex verursachte Modulation des Anteils des an der Basisfläche reflektierten Lichtes wird gemessen und als Maß für die in der Probe absorbierte Laserleistung herangezogen. Obwohl bei dieser Anordnung die gleichen physikalischen Effekte ausgenutzt werden wie bei den bekannten faseroptischen Hydrophonen, können diese zu einer solchen indirekten Messung der absorbierten Laserleistung nicht eingesetzt werden, da sie die Ausbreitung des Laserstrahls beeinflussen würden. Aus diesem Grund wird das Licht seitlich in einen Prismenstumpf eingekoppelt, dessen Basisfläche senkrecht zur Ausbreitungsrichtung des Laserstrahls steht und der für diesen Laserstrahl transparent ist, so dass dieser ungehindert durch den Prismenstumpf hindurch treten kann. Bei der Messanordnung steht außerdem nicht die Messung der Verteilung des Schallfeldes im Vordergrund, da es nur auf die gesamte innerhalb der Probe absorbierte Laserleistung ankommt.

Die Erfindung beruht nun auf der Erkenntnis, dass das in dieser bekannten Messanordnung verwendete Grundkonzept, nämlich als messempfindliche Fläche nicht das Ende einer Faser sondern den beleuchteten Teilbereich eines massiven Körpers zu verwenden, grundsätzlich auch bei der räumlich hochauflösenden Vermessung eines Ultraschallfeldes geeignet ist, da das räumliche Auflösungsvermögen trotz großer Abmessungen des transparenten Körpers allein durch die Größe des beleuchteten Teilbereiches bestimmt ist.

In einer vorteilhaften Ausgestaltung der Erfindung trifft das Licht unter einem Einfallswinkel auf den Flächenbereich auf, der deutlich kleiner ist als der Grenzwinkel der Totalreflexion und insbesondere kleiner als der halbe Grenzwinkel der Totalreflexion ist. Durch die Inzidenz mit einem vom Grenzwinkel der Totalreflexion signifikant abweichenden Einfallswinkel nimmt zwar die Empfindlichkeit gegenüber einer Anordnung mit nahe am Grenzwinkel der Totalreflexion einfallenden Licht ab, von Vorteil ist aber, dass die Messanordnung unempfindlicher gegen geringfügige Änderungen des Auftreffwinkels ist, da die Reflektivität für Einfallswinkel, die deutlich kleiner sind als der Grenzwinkel der Totalreflexion, nahezu unabhängig vom Auftreffwinkel ist. Darüber hinaus ändert sich bei einem solchen Einfallswinkel und insbesondere im Bereich senkrechter Inzidenz (Einfallswinkel 0°) die Reflektivität nahezu linear mit der Brechzahl des fluiden Mediums und somit auch mit dem Schalldruck, so dass die reflektierte Intensität ebenfalls annähernd linear zum Schalldruck ist.

Insbesondere ist die Grenzfläche des Körpers eben und der beleuchtete Flächenbereich ist deutlich kleiner als die Grenzfläche. Mit anderen Worten: Die Grenzfläche ist um ein Vielfaches größer als der beleuchtete Flächenbereich. In einer vorteilhaften Ausgestaltung kann dann der Körper ortsveränderbar relativ zum Weg des sich in ihm zur Grenzfläche ausbreitenden Lichtes angeordnet sein, so dass der beleuchtete Flächenbereich je nach Lage des Körpers an unterschiedlichen Stellen der Grenzfläche positioniert werden kann. Dadurch kann im Falle einer eventuellen Beschädigung der Grenzfläche im beleuchteten Flächenbereich dieser an eine andere Stelle verlagert werden. Bei einem quaderförmigen Körper geschieht dies durch Verschieben parallel zur Grenzfläche. Der Körper kann auch die Gestalt eines Polygons mit einander gegenüberliegenden ebenen Flachseiten aufweisen. In diesem Fall kann eine Variation der Lage des beleuchteten Flächenbereiches an einer Grenzfläche des Körpers durch Drehung des Körpers um eine Symmetrieachse parallel zu diesen Flachseiten erfolgen.

Der optisch transparente Körper hat vorzugsweise eine Brechzahl, die möglichst nahe an der Brechzahl des fluiden Mediums liegt. Dann ist die statische Reflektivität, d. h. die Reflektivität in Abwesenheit eines Ultraschallfeldes minimal und das Signal-Rauschverhältnis maximal.

In einer weiteren bevorzugten Ausgestaltung der Erfindung ist der beleuchtete Flächenbereich wenigstens annähernd kreisscheibenförmig. Auf diese Weise ist gewährleistet, das die Empfindlichkeit des Hydrophons unabhängig von seiner Drehposition um die Ausbreitungsrichtung des Lichtes ist.

Weitere vorteilhafte Ausgestaltungen sind in den weiteren Unteransprüchen wiedergegeben.

Zur weiteren Erläuterung der Erfindung wird auf das Ausführungsbeispiel der Zeichnung verwiesen. Es zeigen:

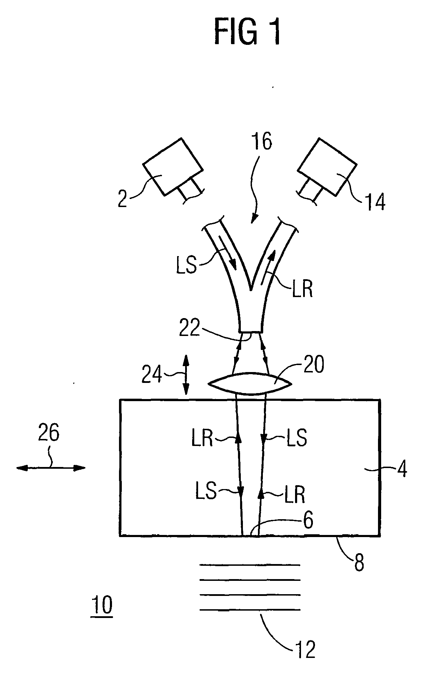

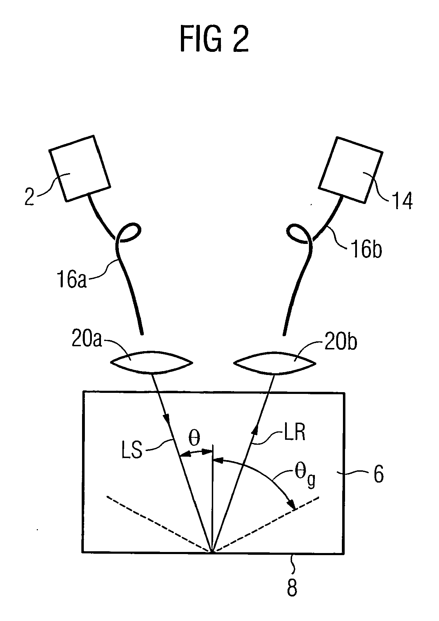

Fig. 1, 2 ein optisches Hydrophon gemäß der Erfindung jeweils in einem Prinzipbild.

Gemäß Figur 1 umfasst das optische Hydrophon eine Lichtquelle 2 zum Erzeugen von Licht LS, im Ausführungsbeispiel eine Laserdiode, das in einen transparenten Körper 4, im Ausführungsbeispiel ein aus Glas (im Ausführungsbeispiel Quarzglas mit einem Brechungsindex n_{K} = 1,45 bei einer Wellenlänge von 800 nm) bestehender annähernd kubischer Block, eingekoppelt wird. Sowohl die Dicke als auch die seitlichen Abmessungen des Körpers 4 liegen im Bereich von 1 mm bis 50 mm. Innerhalb des Körpers 4 breitet sich das gesendete Licht LS frei, d. h. ohne Reflexion an Wänden des Körpers 4 aus und beleuchtet wenigstens annähernd senkrecht, d. h. bei einem Körper 4 aus Glas mit einem Öffnungswinkel kleiner als 10° (in der Figur ist der Öffnungswinkel vergrößert dargestellt) einen kreisscheibenförmigen Flächenbereich 6 einer ebenen Grenzfläche 8. Ein Teil des auf den Flächenbereich 6 auftreffenden Lichtes LS wird dort reflektiert. Die Intensität des reflektierten Lichtes LR hängt bei senkrechter Inzidenz nur ab von der Intensität des auftreffenden Lichtes LS und den Brechungsindizes n_{K} des Körpers 4 und n_{M} des außerhalb des Körpers 4 befindlichen fluiden Mediums 10, im vorliegenden Fall Wasser mit Brechungsindex n_{M} = 1,34 (bei 800 nm).

Eine auf die Grenzfläche 8 einfallende Ultraschallwelle 12 erzeugt eine Modulation des Brechungsindex n_{M} des fluiden Mediums 10 (die durch die Ultraschallwelle erzeugte Modulation des Brechungsindex n_{K} des Körpers 4 ist vernachlässigbar) und somit eine Modulation der Intensität des an der Grenzfläche 8 reflektierten Lichtes LR. Der zeitliche Verlauf der Intensität des reflektierten Lichtes LR wird in einem Lichtempfänger 14, beispielsweise eine Fotodiode, gemessen und ist ein direktes Maß für den zeitlichen Verlauf des Schalldrucks im beleuchteten Flächenbereich 6.

Im Ausführungsbeispiel werden das gesendete Licht LS und das reflektierte Licht LR außerhalb des Körpers 4 in einer Lichtleiteranordnung 16 geführt, wobei zur Auftrennung der Lichtwege ein Y-Koppler 18 vorgesehen ist.

Grundsätzlich ist es jedoch auch möglich, dass sich das Licht 2 zwischen der Lichtquelle 2 und dem Körper 4 bzw. dem Körper 4 und Lichtempfänger 14 frei ausbreitet. Zum Entkoppeln der beiden Lichtwege können dann Strahlteiler eingesetzt werden.

Zum Fokussieren des gesendeten Lichtes LS auf die Grenzfläche 8 ist eine Abbildungsoptik 20 vorgesehen, die die Austrittsapertur 22 des Y-Kopplers 18, die zugleich Eintrittsapertur für das reflektierte Licht LR ist, auf die Grenzfläche 8 abbildet. Die Austrittsapertur 22 beträgt im Ausführungsbeispiel 0,125 mm und kann durch Verstellen der Abbildungsoptik 20 (Pfeil 24) auf 1 mm vergrößert werden.

Die Abbildungsoptik 20, der Y-Koppler 18 oder der Strahlteiler können auch im Inneren des Körpers 4 angeordnet sein, so dass ein kompakter und unempfindlicher Aufbau des Hydrophons möglich ist.

Der Körper 4 kann relativ zur Abbildungsoptik 20 quer zu deren optischer Achse (quer zum Lichtweg bzw. quer zur Normalen des Flächenbereiches 6 oder der Grenzfläche 8) verschiebbar angeordnet sein, wie dies durch den Pfeil 26 veranschaulicht ist. Tritt durch den Ultraschallimpuls oder durch Kavitationsblasen eine Beschädigung der Oberfläche des Körpers 4 in der Nähe des beleuchteten Flächenbereiches 6 auf, so kann der Körper 4 um einige mm verschoben werden, bis der beleuchtete Flächenbereich 6 wieder an einer unbeschädigten Stelle des Körpers 4 zu liegen kommt.

Die Notwendigkeit der Verwendung eines Strahlteilers oder eines Y-Kopplers entfällt, wenn das von der Lichtquelle 2 erzeugte Licht LS unter einem von 0° abweichenden Winkel auf die Grenzfläche 8 auftrifft. In diesem Fall sind einfallendes Licht LS und reflektiertes Licht LR abhängig vom Einfallswinkel und vom Strahldurchmesser nach entsprechender Wegstrecke entkoppelt, d.h. Austrittsapertur und Eintrittsapertur können räumlich getrennt voneinander angeordnet werden, so dass die Lichtleiteranordnung aus getrennte Lichtleitern aufgebaut werden kann. Außerdem kann dann auch die Abbildungsoptik 20 in eine Abbildungsoptik für das gesendete Licht LS und eine Abbildungsoptik für das reflektierte Licht LR räumlich aufgeteilt werden.

Ein solches Ausführungsbeispiel zeigt Fig. 2, gemäß dem das von der Lichtquelle erzeugte Licht LS unter einem von 0° abweichenden Einfallswinkel θ auftrifft, der jedoch deutlich kleiner als der Grenzwinkel θ_{g} der Totalreflexion ist. Deutlich kleiner als der Grenzwinkel θ_{g} der Totalreflexion im Sinne der Erfindung ist ein Einfallswinkel θ, bei dem die Abhängigkeit der Reflektivität vom Einfallswinkel θ nur schwach ist. Dies ist in der Praxis für Einfallswinkel θ der Fall, die insbesondere kleiner als θ_{g}/2, vorzugsweise kleiner als θ_{g}/3 sind. Im vorliegenden Fall - bei n_{K} = 1,45 und n_{M} = 1,34 und einem Genzwinkel θ_{g} der Totalreflektion von 67°- sind dies Einfallswinkel θ < 33° bzw. θ < 22°. In einer praktischen Ausführungsform hat sich ein Einfallswinkel von etwa 10° als besonders geeignet erwiesen.

Aufgrund des größeren Einfallswinkels θ sind die Lichtwege des erzeugten Lichts LS und des reflektierten Lichts LR außerhalb des Körpers 4 räumlich voneinander getrennt, so dass zum Abbilden des aus der Austrittsapertur des Lichtleiters 16a austretenden Lichts LS auf die Grenzfläche 8 und zum Einkoppeln des reflektierten Lichtes LR in die räumlich von der Austrittsapertur getrennte Eintrittsapertur des Lichtleiters 16b voneinander ebenfalls räumlich getrennte Abbildungsoptiken 20a bzw. b verwendet werden können.

## Patentansprüche

1. Verfahren zum Messen der Schalldruckverteilung in einem fluiden Medium (10), insbesondere zum Vermessen eines Ultraschall-Stoßwellenfeldes oder von diagnostischem Ultraschall, unter Ausnutzung der vom Schalldruck abhängigen Modulation des Brechungsindex des fluiden Mediums (10), mit einem optischen Hydrophon umfassend einen optisch transparenten Körper (4) und eine Lichtquelle (2) zum Erzeugen von Licht (LS) und zum Beleuchten eines an einer Grenzfläche (8) zwischen dem Körper (4) und dem fluiden Medium (10) befindlichen Flächenbereiches (6), wobei der optisch transparente Körper (4) einen Brechungsindex aufweist, dessen Abhängigkeit vom Schalldruck vernachlässigbar ist, sowie einem Lichtempfänger (14) zum Messen der Intensität des an diesem Flächenbereich (6) reflektierten Lichtes (LR) als Maß für den Schalldruck, **dadurch gekennzeichnet, dass** der beleuchtete Flächenbereich (6) kleiner ist als die zwischen dem Körper (4) und dem fluiden Medium (10) gebildete Grenzfläche (8) und sich das Licht (LS) innerhalb des Körpers (4) frei ausbreitet, und das Licht (LS) den Flächenbereich (6) mit einem Einfallswinkel (θ) beleuchtet, der kleiner als der halbe Grenzwinkel (θg) der Totalreflektion ist.

2. Verfahren nach Anspruch 1, bei dem zum Führen des Lichtes (LS, LR) eine Lichtleiteranordnung mit räumlich voneinander getrennten Lichtleitern (16a bzw. b) vorgesehen ist.

3. Verfahren nach Anspruch 2, bei dem zum Abbilden des aus dem Lichtleiter (16a) austretenden Lichtes (LS) auf die Grenzfläche (8) und zum Einkoppeln des reflektierten Lichtes (LR) in den Lichtleiter (16b) voneinander räumlich getrennte Abbildungsoptiken (20a, 20b) vorgesehen sind.

4. Verfahren nach Anspruch 1, bei dem das Licht (LS) den Flächenbereich (6) wenigstens annähernd senkrecht beleuchtet.

5. Verfahren nach Anspruch 4, bei dem der beleuchtete Flächenbereich (6) wenigstens annähernd kreisscheibenförmig ist.

6. Verfahren nach Anspruch 4 oder 5, bei dem zum Führen des von der Lichtquelle (2) erzeugten Lichtes (LS) zum Körper (4) und des reflektierten Lichtes (LR) zum Lichtempfänger (14) eine Lichtleiteranordnung (16) vorgesehen ist.

7. Verfahren nach Anspruch 6, bei dem die Lichtleiteranordnung (16) eine Austrittsapertur (22) aufweist, aus der das von der Lichtquelle (2) erzeugte Licht (LS) zum Körper (4) hin austritt, die zugleich Eintrittsapertur für das reflektierte Licht (LR) ist.

8. Verfahren nach Anspruch 7, bei dem die Lichtleiteranordnung (16) einen Y-Koppler (18) aufweist.

9. Verfahren nach einem der vorhergehenden Ansprüche, mit einer Abbildungsoptik (20, 20a) zum Fokussieren des aus der Austrittsapertur (22) austretenden Lichtes (LS) auf den Flächenbereich (6).

10. Verfahren nach Anspruch 9, bei dem die Abbildungsoptik (20, 20a) die Austrittsapertur (22) auf die Grenzfläche (8) abbildet.

11. Verfahren nach einem der vorhergehenden Ansprüche, bei dem die Grenzfläche (8) des Körpers (4) eben ist.

12. Verfahren nach einem der vorhergehenden Ansprüche, bei dem der Körper (4) ortsveränderbar relativ zum Weg des sich in ihm zur Grenzfläche (8) ausbreitenden Lichtes (LS) angeordnet ist.

## Claims

1. Method for measuring the acoustic pressure distribution in a fluid medium (10), in particular for calibrating an ultrasound shock-wave field or diagnostic ultrasound, using the modulation of the refractive index of the fluid medium (10) which is dependent on the acoustic pressure, having an optical hydrophone including an optically transparent body (4) and a light source (2) for generating light (LS) and for illuminating an area (6) located at a boundary surface (8) between the body (4) and the fluid medium (10), with the optically transparent body (4) having a refractive index, the dependency of which on the acoustic pressure is negligible, as well as a light receiver (14) for measuring the intensity of the light (LR) reflected at this area (6) as a measurement of the acoustic pressure, **characterised in that** the illuminated area (6) is smaller than the boundary area (8) formed between the body (4) and the fluid medium (10) and the light (LS) propagates freely within the body (4) and the light illuminates the area (6) with an angle of incidence (θ) that is smaller than half of a critical angle (θ_{g}) of the total reflection.

2. Method according to claim 1, in which an optical fibre arrangement is provided with optical conductors (16a / 16b) which are arranged spatially separated from one another in order to guide the light (LS, LR).

3. Method according to claim 2, in which spatially separated imaging optics (20a, 20b) are provided in order to the project the light (LS) leaving the optical conductor (16a) onto the boundary surface (8) and to inject the reflected light (LR) into the optical conductor (16b).

4. Method according to claim 1, in which the light (LS) illuminates the area (6) in an at least approximately vertical manner.

5. Method according to claim 4, in which the illuminated area (6) is at least approximately a type of circular disk.

6. Method according to claim 4 or 5, in which an optical fibre arrangement (16) is provided in order to guide the light (LS) generated by the light source (2) towards the body (4) and to guide the reflected light (LR) towards the light receiver (14).

7. Method according to claim 6, in which the optical fibre arrangement (16) has an exit aperture (22), from which the light (LS) generated by the light source (2) exits towards the body (4), which similarly serves as an entrance aperture for the reflected light (LR).

8. Method according to claim 7, in which the optical fibre arrangement (16) has a y-coupler (18).

9. Method according to one of the preceding claims, having an imaging optic (20, 20a) for focussing the light (LS) leaving the exit aperture (22) onto the area (6).

10. Method according to claim 9, in which the imaging optic (20, 20a) projects the exit aperture (22) onto the area (8).

11. Method according to one of the preceding claims, in which the area (8) of the body (4) is flat.

12. Method according to one of the preceding claims, in which the body (4) is arranged in a spatially variable manner relative to the path of the light (LS) propagating therein towards the boundary surface (8).

## Revendications

1. Procédé de mesure de la répartition de la pression acoustique dans un milieu (10) fluide, notamment de mesure d'un champ d'onde de choc ultrasonore ou d'ultrason de diagnostic, en utilisant la modulation de l'indice de réfraction du milieu (10) fluide en fonction de la pression acoustique, comprenant un hydrophone optique comportant une pièce (4) transparente du point de vue optique et une source (2) lumineuse pour produire de la lumière (LS) et pour éclairer une partie (6) de surface se trouvant à une interface (8) entre la pièce (4) et le milieu (10) fluide, la pièce (4) transparente du point de vue optique ayant un indice de réfraction dont la variation en fonction de la pression acoustique est négligeable, ainsi qu'un récepteur (14) de lumière pour mesurer l'intensité de la lumière (LR) réfléchie sur cette partie (6) de surface en tant que mesure de la pression acoustique, **caractérisé en ce que** la partie (6) de surface éclairée est plus petite que l'interface (8) formée entre la pièce (4) et le milieu (10) fluide et la lumière (LS) se propage librement au sein de la pièce (4) et la lumière (LS) éclaire la partie (6) de surface sous un angle (θ) d'incidence qui est plus petit que le demi-angle (θ_{g}) limite de la réflexion totale.

2. Procédé suivant la revendication 1, dans lequel, pour guider la lumière (LS, LR), il est prévu un dispositif de fibres optiques ayant des fibres (16a ou 16b) optiques séparées l'une de l'autre dans l'espace.

3. Procédé suivant la revendication 2, dans lequel, pour projeter sur l'interface (8) la lumière (LS) sortant de la fibre (16a) optique et pour injecter de la lumière (LR) réfléchie dans la fibre (16b) optique, il est prévu des optiques (20a, 20b) de reproduction séparées l'une de l'autre dans l'espace.

4. Procédé suivant la revendication 1, dans lequel la lumière (LS) éclaire à peu près au moins sensiblement perpendiculairement la partie (6) de surface.

5. Procédé suivant la revendication 1, dans lequel la partie (6) de surface éclairée est au moins à peu près en forme de disque circulaire.

6. Procédé suivant la revendication 4 ou 5, dans lequel pour guider la lumière (LS) produite par la source (2) lumineuse vers la pièce (4) et la lumière (LR) réfléchie vers le récepteur (14) de lumière, il est prévu un dispositif (16) à fibres optiques.

7. Procédé suivant la revendication 6, dans lequel le dispositif (16) à fibres optiques a une ouverture (22) de sortie de laquelle la lumière (LS) produite par la source (2) lumineuse sort vers la pièce (4), ouverture qui est en même temps une ouverture d'entrée de la lumière (LR) réfléchie.

8. Procédé suivant la revendication 7, dans lequel le dispositif (16) à fibres optiques comporte un coupleur (18) en Y.

9. Procédé suivant l'une des revendications précédentes comprenant une optique (20, 20a) de reproduction pour focaliser sur la partie (6) de surface la lumière (LS) sortant de l'ouverture (22) de sortie.

10. Procédé suivant la revendication 9, dans lequel l'optique (20, 20a) de reproduction reproduit l'ouverture (22) de sortie sur l'interface (8).

11. Procédé suivant l'une des revendications précédentes, dans lequel l'interface (8) de la pièce (4) est plane.

12. Procédé suivant l'une des revendications précédentes, dans lequel la pièce (4) est disposée de manière à ce que son emplacement puisse être modifié par rapport au trajet de la lumière (LS) s'y propageant vers l'interface (8).
